# EUROPEAN PATENT SPECIFICATION

(11) **EP 1 940 447 B1**
(45) Date of publication and mention of the grant of the patent: **27.11.2013**
(21) Application number: 06793581.7
(22) Date of filing: 18.09.2006
(51) Int. Cl.: A61K 38/18, A61K 47/48, A61P 25/28, A61P 25/16, A61P 25/18

(54) **TREATMENT OF NEURODEGENERATIVE DISORDERS**
BEHANDLUNG VON NEURODEGENERATIVEN STÖRUNGEN
TRAITEMENT DES MALADIES NEURODÉGÉNÉRATIVES

(30) Priority: 28.09.2005 EP 05108946
(43) Date of publication of application: 09.07.2008
(73) Proprietor: F.Hoffmann-La Roche AG, 4070 Basel (CH)
(72) Inventor: HASELBECK, Anton, 82362 Weilheim (DE); HERTING, Frank, 82377 Penzberg (DE); HUWYLER, Joerg, CH-4117 Burg im Leimental (CH); JARSCH, Michael, 83670 Bad Heilbrunn (DE)
(74) Representative: Salud, Carlos E.
(86) International application number: PCT/EP2006/066438
(87) International publication number: WO 2007/039436

(56) References cited:
- EP-A- 1 525 889
- EP-A2- 1 064 951
- WO-A-00/61164
- WO-A-2004/047858
- WO-A-2005/058347
- WO-A2-02/49673
- WO-A2-2004/112693
- US-A1- 2004 092 444
- ERBAYKTAR, S. ET AL.: "Asialoerythropoietin is a nonerythropoietic cytokine with broad neuroprotective activity in vivo" PNAS, vol. 100, no. 11, 27 May 2003 (2003-05-27), pages 6741-6746, XP002415679

### SEQUENCE LISTING

<110> F. Hoffmann-La Roche AG
<120> treatment of neurodegenerative disorders
<130> 23251
<160> 3
<170> PatentIn version 3.3
<210> 1
   <211> 165
   <212> PRT
   <213> Homo sapiens
<400> 1
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp
                   165
<210> 2
   <211> 166
   <212> PRT
   <213> Homo sapiens
<400> 2
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp Arg
                   165
<210> 3
   <211> 28
   <212> PRT
   <213> Homo sapiens
<400> 3
   Ser Ser Ser Ser Lys Ala Pro Pro Pro Ser Leu Pro Ser Pro Ser Arg
   1               5                   10                  15
   Leu Pro Gly Pro Ser Asp Thr Pro Ile Leu Pro Gln
                       20                  25

## Description

The present invention relates to a method of treating neurodegenerative disorders of the brain and spinal cord using a novel erythropoietic agent (NEA).

The bioavailability of commercially available protein therapeutics such as human erythropoietin (EPO) is limited by their short plasma half-life and susceptibility to protease degradation. These shortcomings prevent them from attaining maximum clinical potency. Novel erythropoietic agents have been developed through chemical modification of EPO and analogs thereof. These novel agents provide potent and prolonged erythropoietic activity allowing optimal anemia management in patients with kidney disease and in AIDS and cancer patients undergoing chemotherapy.

The present invention relates to a method of treating neurodegenerative disorders of the brain and spinal cord by administering to a patient in need of such therapy a therapeutically effective amount of a novel erythropoietic agent (NEA) that is a chemically modified human erythropoietin or chemically modified human erythropoietin analog comprising covalently integrated poly(ethylene glycol) groups having particular molecular weight and linker structure.

Description of the Figures:
Figure 1 depicts the concentration of EPO and an NEA of the invention in the serum of rats 2 and 6 hours after injection.
Figure 2 depicts the concentration of EPO and an NEA of the invention in the liquor of rats 2 and 6 hours after injection.
Figure 3 depicts the concentration of EPO and an NEA of the invention in the liquor as well as in the serum of rats 2 and 6 hours after injection.

Specifically, the NEAs used in this invention are chemically modified erythropoietic molecules having preferably at least one free amino group and comprising an erythropoietin moiety selected from the group consisting of human erythropoietin and analogs thereof which have the sequence of human erythropoietin modified by the addition of from 1 to 6 glycosylation sites or a rearrangement of at least one glycosylation site; said erythropoietin moiety being covalently linked to "n" poly(ethylene glycol) groups of the formula -CO-(CH₂)ₓ-(OCH₂CH₂)ₘ-OR with the-CO (i.e. carbonyl) of each poly(ethylene glycol) group forming an amide bond with one of said amino groups; wherein R is lower alkyl; x is 2 or 3; m is from about 450 to about 900; n is from 1 to 3; and n and m are chosen so that the molecular weight of the resulting NEA subtracted by the molecular weight of the unmodified erythropoietin moiety equals from about 20 kilodaltons to about 100 kilodaltons. Such NEAs are described, for example, in US Pat. No. 6,583,272, which to the extent necessary, is herein incorporated by reference.

The NEAs useful in this invention are biochemically and functionally distinct from EPO. Together, *in vivo* and *in vitro* data indicate that these NEAs exhibit substantially lower binding affinity to the EPO receptor and dissociate more quickly, compared with EPO. Compared to human erythropoietin (hEPO), these NEAs exhibit distinct, advantageous clinical properties, including increased circulating half-life and plasma residence time, decreased clearance, and increased clinical activity *in vivo.*

Some of the above observations relating to distinct properties of the NEAs of the invention possibly may be explained by a novel mode of action. Rapid dissociation from the erythropoietin receptor ("EPO-R") together with an extended serum half-life may result in an enhanced and sustained erythropoietic effect through multiple interactions with the receptor. For steric reasons, these multiple interactions might be sufficient to induce the signal cascade of the EPO-R but are not tight enough to result in such a strong binding that the receptor/molecule complex is internalized and degraded. Statistically, only a certain percentage of the molecules might commit such a tight binding. In total, this mode of action would lead to the effect that one molecule would activate more than one receptor before being degraded.

Importantly, the advantageous properties of these NEAs allow for decreased frequency of administration and more stable control of hemoglobin, permitting optimal management of anemia in patients with kidney disease and patients with AIDS or cancer undergoing chemotherapy. These advantages are expected to result in improved treatment outcomes as well as improved patient quality of life.

Naturally occurring human erythropoietin (hEPO) is produced in different tissues of the body (e.g. kidneys, brain et.) and is the humoral plasma factor which inter alia stimulates red blood cell production (Carnot, P and Deflandre, C (1906) C.R. Acad. Sci. 143: 432; Erslev, AJ (1953 Blood 8: 349; Reissmann, KR (1950) Blood 5: 372; Jacobson, LO, Goldwasser, E, Freid, W and Plzak, LF (1957) Nature 179: 6331-4). Naturally occurring EPO stimulates the division and differentiation of committed erythroid progenitors in the bone marrow and exerts its biological activity by binding to receptors on erythroid precursors (Krantz, BS (1991) Blood 77: 419).

In addition to the use of EPO to treat anemia, recently, this molecule is also postulated to provide neuro and myocardial protective effects. See review article W. Jelkmann and K. Wagner, Ann. Hematol 83:673-686 (2004).

This invention provides for the use of the NEAs of the invention for the treatment of neurodegenerative disorders of the brain and the spinal cord by introducing the NEA in the blood circuit. This invention is based on the finding that despite their relatively large size, the NEAs of this invention are also capable of crossing the blood brain barrier to serve as neuroprotective agents for neurons found in the brain and the spinal cord. The distinct, superior clinical properties that these NEAs exhibit in other settings as described above are expected also to provide a substantial therapeutic advantage when used to treat neurodegenerative disorders, as compared to therapy with EPO.

Erythropoietin has been manufactured biosynthetically using recombinant DNA technology (Egrie, JC, Strickland, TW, Lane, J et al. (1986) Immunobiol. 72: 213-224) and is the product of a cloned human EPO gene inserted into and expressed in the ovarian tissue cells of the Chinese hamster (CHO cells). The primary structure of the predominant, fully processed form of hEPO is illustrated in SEQ ID NO:1. There are two disulfide bridges between Cys⁷-Cys¹⁶¹ and Cys²⁹-Cys³³. The molecular weight of the polypeptide chain of EPO without the sugar moieties is 18,236 Da. In the intact EPO molecule, approximately 40% of the molecular weight is accounted for by the carbohydrate groups that glycosylate the protein at Glycosylation sites on the protein (Sasaki, H, Bothner, B, Dell, A and Fukuda, M (1987) J. Biol. Chem. 262: 12059).

The term "erythropoietin" or "EPO" refers to a glycosylated protein, having the amino acid sequence set out in (SEQ ID NO: 1) or (SEQ ID NO: 2) or an amino acid sequence substantially homologous thereto, whose biological properties can be related to the stimulation of red blood cell production and the stimulation of the division and differentiation of committed erythroid progenitors in the bone marrow. Furthermore, "erythropoietin" refers to a glycosylated protein showing at least one of the biological properties or binding affinities known in the state of the art. Thus, molecules are comprised exhibiting neuroprotective effects only. As used herein, these terms include such proteins modified deliberately, as for example, by site directed mutagenesis or accidentally through mutations. These terms also include analogs having from 1 to 6 additional sites for glycosylation, analogs having at least one additional amino acid at the carboxy terminal end of the glycoprotein, wherein the additional amino acid includes at least one glycosylation site, and analogs having an amino acid sequence which includes a rearrangement of at least one site for glycosylation. These terms include both natural and recombinant produced human erythropoietin.

EPO binds to specific transmembrane receptors (EPO-R). The functional human EPO-R is a member of the cytokine class I receptor superfamily and presents as a homodimer of two identical glycoprotein chains of 484 amino acids. Each chain comprises an extracellular domain, a hydrophobic transmembrane sequence, and a cytoplasmic domain to which the protein tyrosine kinase JAK2 is affiliated. Unmodified EPO binds to the receptor subunits, whereby the dissociation constants for the two binding sites differ greatly. The binding of EPO with the receptor leads to a conformational change and a tighter connection of the two EPO-R subunits which leads to an autophosphorylation of the two JAK molecules which results in a complex signalling cascade. It has been shown that the EPO-induced signalling pathway returns to nearly basal levels after 30-60 min of stimulation. The effect of EPO is terminated by the action of the hemopoietic cell phosphatase (HCP) causing the internalization and degradation of the EPO/EPO-R complex.

Recently it has been shown that EPO is a more pleiotropic survival growth factor than initially thought. It is believed that EPO has neurotrophic and neuroprotective (Cerami A et al. (2002) Nephrol. Dial. Transplant. 17: 8-12; Chong, ZZ et al. (2003) Curr. Drug Targets Cardiovasc. Haematol. Disord. 3: 141-154; Jumbe, NL (2002) Oncology 16: 91-107; Marti, HH et al. (2000) News Physiol. Sci. 15: 225-229), vascular (Masuda, Set al. (1999) Int. J. Hematol. 70: 1-6; Smith, KJ et al. (2003) Cardiovasc. Res. 59: 538-548), and cardioprotective functions (Smith, KJ et al. (2003) Cardiovasc. Res. 59: 538-548; Parsa, CJ et al. (2003) J. Clin. Invest. 112: 999-1007). It has been shown that EPO-R is present in distinct areas of rodent and mammalian brain (Digicaylioglu, M et al. (1995) Proc. Natl. Acad. Sci. USA 92: 3717-3720; Li, Yet al. (1996) Pediatr. Res. 40: 376-380; Marti, HH et al. (1996) Eur. J. Neurosci. 8: 666-676). EPO binding sites were mainly located in the hippocampus, capsula interna, cortex, and midbrain of mice (Digicaylioglu, M et al. (1995) Proc. Natl. Acad. Sci. USA 92: 3717-3720). Furthermore it is known that EPO stimulates the proliferation and differentiation of neuronal stem and progenitor cells (Shingo, T et al. (2001) J. Neurosci. 21: 9733-9743; Studer, Let al. (2000) J. Neurosci. 20: 7377-7383).

The neuroprotective effect of EPO can be traced back to the primary importance of the PI-3K/Akt pathway in the neuroprotective action of EPO by maintaining mitochondrial membrane potential in anoxic primary hippocampal neuronal cell cultures (Chong, ZZ et al. (2003) Circulation 106: 2973-2979). Destabilization of the mitochondrial membrane potential leads to the release of cytochrome C, which activates the caspases 8, 1, and 3 that promote DNA fragmentation.

An *in vivo* neuroprotective effect of EPO in the brain was first provided by the group of Sasaki in 1998 (Sadamoto, Yet al. (1998) Biochem. Biophys. Res. Commun. 253: 26-32; Sakanak, M et al. (1998) Proc. Natl. Acad. Sci. USA 95: 4635-4640) performed in Mongolian gerbils. It was shown that the infusion of EPO into the lateral ventricles prevents ischemia-induced learning disability and rescues hippocampal CA1 neurons from death. Similar experiments with rats have shown a reduction of ischemia-induced place navigation disability, cortical infarction, and thalamic degeneration (Sadamoto, Yet al. (1998) Biochem. Biophys. Res. Commun. 253: 26-32). Furthermore, the known protective effect of hypoxic preconditioning is significantly reduced in mice when EPO signalling is locally blocked by infusion of soluble EPO-R into the cerebral ventricle (Prass, K et al. (2003) Stroke 34: 1981-1986).

It was earlier assumed that systemically administered EPO would not enter the brain because of the blood-brain barrier (Junk, AK et al. (2002) Proc. Natl. Acad. Sci USA 99: 10659-10664; Juul, SE et al. (1999) Pediatr. Res. 46: 543-547).The blood brain barrier (BBB) separates the brain as well as the cerebrospinal fluid (CSF, liquor) from the blood and regulates the exchange of substances between the blood and the brain. As used herein, the term "BBB" comprises the blood brain barrier as well as the blood-CSF barrier. It is comprised chiefly of brain capillaries, choroids plexus cuboidal epithelium, and the arachnoid membrane. All BBB sites are characterized by the presence of tight junctions between contiguous cells, the absence of endothelial pores, and a paucity of pinocytic vesicles. Further, brain capillaries contain a several-fold increase in the numerical density of endothelial mitochondria as compared with capillaries from other regions of the body. The cells constituting the BBB effectively function as a continuous cell layer, permitting solute exchange primarily by the transcellular route only. Thus, lipid soluble solutes easily penetrate the BBB while electrolytes, lipid-insoluble nonelectrolytes, and proteins enter the brain from blood more slowly than they enter non-nervous tissues. This barrier function helps to protect the brain from harmful substances.

There are four basic mechanisms by which solute molecules move across membranes. (1) Simple diffusion, (2) facilitated diffusion, (3) simple diffusion through an aqueous channel, and (4) activated transport through a protein carrier. Paracellular diffusion does not occur to any great extent at the BBB, due to the tight junctions. In case of transcellular diffusion, the general rule is the higher the lipophilicity of a substance, the greater the diffusion into the brain. Glucose, alcohol and other small molecules just get in the brain by diffusion. Most proteins usually need to use an activated transport.

The blood brain barrier can be "opened" by certain solutions such as the intra-arterial injection of hypertonic mannitol. Mannitol is thought to open the blood brain barrier through osmosis by shrinking the endothelial cells.

The CSF is located within the ventricles, spinal canal, and subarachnoid spaces. The principle sources of CSF are the choroids plexi of the lateral, third and fourth ventricles, and the volume varies between 10-20% of the brain weight. The volume of CSF in humans is 140-150 ml with a turnover of 5 h for humans (1h for rat). CSF moves within the ventricles and subarachnoid spaces under the influence of hydrostatic pressure generated by its production. CSF cushions the brain, regulates brain extracellular fluid, allows for distribution of neuroactive substances, and is the sink that collects the waste products produced by the brain.

Jumbe (Jumbe NL (2002) Oncology 16: 91-107) has shown that the cerebrospinal fluid to serum concentration ratios of rats administered recombinant human EPO intravenously (500 U/kg) was about 1x10⁻³. Similar is true for the administration of darbepoetin alpha with 25 µg/kg. The calculated mean area under the concentration-time curve (AUC₀₋₈), by noncompartemental analysis, was 340 mU h/ml for recombinant human EPO (rhEPO) and 3.6 ng h/ml for darbepoetin alfa in cerebrospinal fluid vs 370,000 mU h/ml and 4500 ng h/ml in serum, respectively.

With respect to stroke experiments it has been shown that the systemic administration of high doses of rhEPO to test animals reduce the volume of infarction 24 h after middle cerebral artery occlusion (Siren, ALet al. (2001) Proc. Natl. Acad. Sci USA 98: 4044-4049), reduces mortality rate (Buemi, M et al. (2000) Eur. J. Pharmacol. 392: 31-34), prevents neuronal damage (Alafaci, C et al. (2000) Eur. J. Pharmacol. 406: 219-225), increases cerebral blood flow (Grasso, G (2001) J. Neurosurg. Sci. 45: 7-14), and reduces neurologic deficits (Grasso, G et al. (2002) J. Neurosurg. 96: 565-570). EPO furthermore prevents motor neuron apoptosis and neurologic disability (Cerami, A et al. (2002) Nephrol. Dial. Transplant 17: 8-12), improves recovery of motor function (Gorio, A et al. (2002) Proc. Natl. Acad. Sci. USA 99: 9450-9455), and reduces the inflammatory reaction in hypoxic brain (Villa, P et al. (2003) J. Exp. Med. 198: 971-975).

Furthermore, the use of erythropoietin in Multiple Sclerosis (MS) is currently under consideration. MS is an inflammatory disease of the Central Nervous System (CNS), which is the brain and spinal cord. In people affected by MS, patches of damage called plaques or lesions appear in seemingly random areas of the CNS white matter. At the site of a lesion, a nerve insulating material, called myelin, is lost probably during an autoimmune inflammation. The myelin sheath gets stripped from the axons in a process known as demyelination. The myelin sheath is formed in the CNS by certain parts of oligodendrocytes. Until now it is not clear what causes MS. Different theories have been proposed, e.g. autoimmunity, pathogen mediated, genetic components, biochemical events *in utero,* damage of the blood brain barrier, diet and vitamin deficiencies, allergic reaction and others. Furthermore, it is discussed, that the inflammation also harms the axonal membrane. So far there is no curative treatment available for MS. However, a number of medications can be used to treat the disease symptomatically. For example, corticosteroids, a number of immunosuppressive drugs and interferon beta can be administered.

Diem et al. (Brain (2005), 128: 375-85) describe a combined steroid treatment with the application of EPO to target inflammatory as well as neurodegenerative aspects. Thus, methylprednisolone and erythropoietin are used successfully as a combined therapy in a model of MS.

Experiments in human done by Ehrenreich et al. (Ehrenreich, H et al. (2002) Mol. Med. 8: 495-505) with stroke patients have shown that there is a strong trend for reduction on infarct size in the rhEPO treated patients associated with a marked neurological recovery and clinical outcome 1 month after stroke. The patients received rhEPO intravenously (3.3x10⁴ U) once daily for the first days after stroke. The mean concentration of EPO in the cerebral spinal fluid of the patients increased to 17 U/l (normal value is about 1 U/l). Serum levels on the patients approximated to 5,000 U/l 3 h after infusion (normal serum levels are about 15 U/l). Furthermore, EPO might be also successfully used to reduce reperfusion injuries of the encompassing region of the acute stroke.

It is known that EPO and EPO-R are expressed in the human and rodent brain tissue (Sirén AL et al. (2001) Acta Neuropathologica 101: 271-276), are hypoxia-inducible (Jelkmann, W (1994) Clin. Investig. 72: 3-10), and have demonstrated remarkable neuroprotective potential (Bernaudin, M et al. (1999) J. Cereb. Blood How Metab. 19: 643-651; Genc, S et al. (2001) Neurosci. Lett. 298: 139-141). In the adult human brain, only a weak expression of EPO and its receptor has been reported in neurons and astrocytes (Sirén AL et al. (2001) Acta Neuropathologica 101: 271-276). Anyway, in human brains after ischemia and/or hypoxia EPO was seen in vascular tissue and inflammatory cells, EPO-R in blood vessels and neuronal and astrocytic processes within the infarcts and the peri-infarct zone. In older ischemic infarcts EPO and EPO-R were strongest in reactive glia. The net effect of EPO-R stimulation in the target cell is proliferation, inhibition of apoptosis and, in the case of erythroblasts, differentiation.

It has been assumed that the BBB effectively excludes large glycosylated molecules such as EPO. Although in the classic view the BBB is considered to be impermeable to large molecules, studies have shown that some large molecules can be specifically transported into the brain across the capillary endothelium to affect brain function. This takes place via binding to receptors present on the luminal surfaces of the endothelial cells. This initiates endocytosis, followed by translocation across the BBB. Since EPO-R is expressed at brain capillaries it is assumed that the transport of EPO through the BBB functions via receptor mediated transport.

Notably, the serum concentrations of EPO required for tissue protection are higher than those required for erythropoiesis. One reason for this is that the receptor for tissue protection exhibits a lower affinity (approximately 1000-fold) as compared with erythroid progenitors (Masuda, S et al. (1993) J. Biol. Chem. 268: 11208-11216). Another reason may be the presence of the BBB. Preclinical data suggest that the minimum therapeutic level of EPO needed for protection against tissue injury appears to be in the order of 300-500 mlU/kg body weight. Units of EPO are defined as the amount of EPO inducing the same erythropoietic reaction in rats like 15 µmol CoCl₂(cobalt chloride).Briefly, it is therefore known that EPO has a neuroprotective effect on neurons of the brain and the spinal cord. However, the potential use of EPO for such therapy is limited by the need for substantially high therapeutic levels which are required to achieve such an effect. A new Erythropoesis Stimulating Factor (ESA) having an improved half-life and crossing the BBB would be preferred, especially if such ESA can be administered in a relatively low starting concentration in the blood circuit to avoid negative side-effects.

Document WO 02/49673 refers to methods and compositions for protecting or enhancing excitable tissue function in mammals by systemic administration of an erythropoietin receptor modulator, such as erythropoietin, which signals via an EPO-activated receptor to modulate the function of excitable tissue.

Document EP 1525889 refers to a liquid pharmaceutical composition comprising an erythropoietin protein, a multiple charged inorganic anion in a pharmaceutically acceptable buffer suitable to keep the solution pH in the range from about 5.5 to about 7.0, and optionally one or more pharmaceutically acceptable excipients.

The problems of the state of the art are solved by the use of an NEA of the invention comprising covalently integrated poly(ethylene glycol) groups having particular molecular weight and linker structure as depicted in the claims as attached. In particular, this NEA is used for the production of a medicament for the treatment of neurodegenerative disorders of the brain and the spinal cord by introducing the medicament in the blood circuit. In a preferred embodiment said NEA is a chemically modified erythropoietic molecule having at least one free amino group and comprising an erythropoietin moiety selected from the group consisting of human erythropoietin and analogs thereof which have sequence of human erythropoietin modified by the addition of from 1 to 6 glycosylation sites or a rearrangement of at least one glycosylation site; said erythropoietin moiety being covalently linked to "n" poly(ethylene glycol) groups of the formula -CO-(CH₂)ₓ-(OCH₂CH₂)ₘ-OR with the -CO (i.e. carbonyl) of each poly(ethylene glycol) group forming an amide bond with one of said amino groups; wherein R is lower alkyl; x is 2 or 3; m is from about 450 to about 900; n is from 1 to 3; and n and m are chosen so that the molecular weight of the resulting NEA subtracted by the molecular weight of the unmodified erythropoietin glycoprotein is from about 20 kilodaltons to about 100 kilodaltons.

Preferably, the NEA is of the formula:

P-[NHCO-(CH₂)ₓ-(OCH₂CH₂)ₘ-OR]ₙ (I)

wherein x, m, n and R are as defined in claim 1, and P is the residue of the erythropoietin moiety without the n amino group(s) which form amide linkage(s) with the poly(ethylene glycol) group(s). Within above depicted form, R is most preferably methyl, m is from about 650 to about 750, and n is 1.

Most preferably, the NEA used in the method of the invention has the formula

[CH₃O(CH₁CH₂O)ₘCH₂CH₂CH₂CO-NH]ₙ-P

wherein m is from 650 to 750, n is 1 and P is the residue of an erythropoietin moiety.

Preferably the erythropoietin moiety is a human erythropoietin glycoprotein, which can be expressed by endogenous gene activation, and has the amino acid sequence of SEQ ID NO:1.

Alternatively the erythropoietin moiety has the sequence of human erythropoietin modified by the addition of from I to 6 glycosylation sites.

In another preferred embodiment the neurodegenerative disorders of the brain and the spinal cord treatable by the method of the invention are related to an acute event selected from stroke, TBI (Traumatic Brain Injury) or spinal cord injury. Furthermore, the neurodegenerative disorders of the brain and the spinal cord can be related to a chronic treatment comprising stroke, schizophrenia, Alzheimer's disease, Huntington's disease, dementia, FXTAS (fragile X-associated tremor/ataxia syndrome), Parkinson's disease, spongiform encephalopathy, multiple sclerosis, and neurodegeneration associated with bacterial or viral infections.

In the current method, the NEAis administered in an amount sufficient to treat or ameliorate neurogenerative disorders (a "therapeutically effective amount"). The NEA can be administered to patients by conventional methods used for EPO therapy. The exact amount of NEA depends on the exact type of condition being treated, the condition of the patient being treated, as well as the other ingredients in the composition. The quantity in µg relates to the respective erythropoietin (that is protein) moiety only. Preferably, a patient is administered from about 0.1 to about 100 µg per kg body weight of an ESA of the invention, preferably from about 1 to about 10 µg per kg body weight once weekly.

If necessary, the NEA may be administered more frequently. However, the NEA used according to the invention may also be administered every two weeks, every three weeks or once a month or even in longer time intervals depending on the diseases treated and the kind of administration. The pharmaceutical compositions containing the conjugate may be formulated at a strength effective for administration by various means to a human patient experiencing neurodegenerative disorders characterized by the death of neurons. Average therapeutically effective amounts of the conjugate may vary and in particular should be based upon the recommendations and prescription of a qualified physician.

The specific activity of NEAs in accordance with this invention can be determined by various assays known in the art. The biological activity of the purified NEA of this invention is such that administration of the NEA e.g. by injection, to human patients results in the protection of neurons of the brain and the spinal cord.

The pharmaceutical preparations of the invention include pharmaceutical compositions suitable for injection that are formulated with a pharmaceutically acceptable carrier or vehicle. The preparation of such pharmaceutical compositions is known in the art. See, for example, US 2002/0037841 A1 (corresponding to WO 01/87329), which US Publication is herein incorporated by reference. Pharmaceutically acceptable carriers for formulating the products of the invention include human serum albumin, human plasma proteins, and the like.

Furthermore, the use of spray dried preparations of the composition may be desirable with or without adding any stabilizers or filling material.

The ESAs used in the present invention may be formulated in 10 mM sodium/potassium phosphate buffer at pH 7 containing a tonicity agent, e.g. 132 mM sodium chloride. Optionally the pharmaceutical composition may contain a preservative. The pharmaceutical composition may contain different amounts of erythropoietin protein, e.g. 10 - 1000 µg/ml. preferably 50 µg or 400 µg.

The NEA will be preferably introduced in the blood circuit by injection, dermal patch, subcutaneous deposit or inhalation.

Preferably the NEA will be administered to an individual at a dose of from about 25 µg to about 500 µg/day for up to two weeks with acute cases of neurodegeneration or by applying from about 25 µg to about 1,000 µg/week with chronic treatment of neurodegenerative diseases. The administration in the latter case can also be extended up to once an application every month or even in longer time frames, depending on the type of application and type of disease. In a preferred embodiment, the NEA will be applied with about 165 µg /day up to one week in acute cases or with about 200 µg/week in chronic cases.

Furthermore, the invention concerns a kit comprising an NEA useful according to the aforementioned uses and a substance improving the penetrability of the blood brain barrier and the substance improving the penetrability of the blood brain barrier is mannitol.

Human erythropoietin and analogous proteins as defined above can be expressed by endogenous gene activation. Preferred human erythropoietin glycoproteins are those of SEQ ID NO:1 and SEQ ID NO:2, most preferably those of SEQ ID NO:1.

Further, P may be selected from the group consisting of residues of human erythropoietin and analogs thereof having from 1 to 6 additional sites for glycosylation. As set out in detail below, the preparation and purification of EPO are well known in the art. By EPO is meant the natural or recombinant protein, preferably human, as obtained from any conventional source such as tissues, protein synthesis, cell culture with natural or recombinant cells. Any protein having the activity of EPO, such as muteins or otherwise modified proteins, is encompassed. "Any activity" is this respect also includes the binding specificity to the EPO receptor presented on neuronal cells only. Thus, NEA derivatives according to this invention not showing erythropoietic activity are included. Recombinant EPO may be prepared via expression in CHO-, BHK- or HeLa cell lines, by recombinant DNA technology or by endogenous gene activation. Expression of proteins, including EPO, by endogenous gene activation is well known in the art and is disclosed, for example in U.S. Patents Nos. 5,733,761, 5,641,670, and 5,733,746, and international patent publication Nos. WO 93/09222, WO 94/12650, WO 95/31560, WO 90/11354, WO 91/06667 and WO 91/09955, the contents of each of which are incorporated herein by reference. The preferred EPO species for the preparation of erythropoietin glycoprotein products are human EPO species. More preferably, the EPO species is the human EPO having the amino acid sequence set out in SEQ ID NO:1 or SEQ ID NO:2, more preferably the amino acid sequence SEQ ID NO:1.

Furthermore, P may be the residue of a glycoprotein analog having from 1 to 6 additional sites for glycosylation. Glycosylation of a protein, with one or more oligosaccharide groups, occurs at specific locations along a polypeptide backbone and greatly affects the physical properties of the protein such as protein stability, secretion, subcellular localization, and biological activity. Glycosylation is usually of two types. O-linked oligosaccharides are attached to serine or threonine residues and N-linked oligosaccharides are attached to asparagine residues. One type of oligosaccharide found on both N-linked and O-linked oligosaccharides is N-acetylneuraminic acid (sialic acid), which is a family of amino sugars containing 9 or more carbon atoms. Sialic acid is usually the terminal residue on both N-linked and O-linked oligosaccharides and, because it bears a negative charge, confers acidic properties to the glycoprotein. Human erythropoietin, having 165 amino acids, contains three N-linked and one O-linked oligosaccharide chains which comprise about 40% of the total molecular weight of the glycoprotein. N-linked glycosylation occurs at asparagine residues located at positions 24, 38, and 83 and O-linked glycosylation occurs at a serine residue located at position 126. The oligosaccharide chains are modified with terminal sialic acid residues. Enzymatic removal of all sialic acid residues from the glycosylated erythropoietin results in loss of *in vivo* activity but not *in vitro* activity because sialylation of erythropoietin prevents its binding, and subsequent clearance, by hepatic binding protein.

Glycoproteins used in the chemical synthesis of NEAs of the present invention include analogs of human erythropoietin with one or more changes in the amino acid sequence of human erythropoietin which result in an increase in the number of sites for sialic acid attachment. These glycoprotein analogs may be generated by site-directed mutagenesis having additions, deletions, or substitutions of amino acid residues that increase or alter sites that are available for glycosylation. Glycoprotein analogs having levels of sialic acid greater than those found in human erythropoietin are generated by adding glycosylation sites which do not perturb the secondary or tertiary conformation required for biological activity. Glycoproteins used in the chemical synthesis of NEAs of the present invention also include analogs having increased levels of carbohydrate attachment at a glycosylation site which usually involve the substitution of one or more amino acids in close proximity to an N-linked or O-linked site. Glycoproteins used in the chemical synthesis of NEAs of the present invention also include analogs having one or more amino acids extending from the carboxy terminal end of erythropoietin and providing at least one additional carbohydrate site. Glycoproteins used in the chemical synthesis of NEAs of the present invention also include analogs having an amino acid sequence which includes a rearrangement of at least one site for glycosylation. Such a rearrangement of glycosylation site involves the deletion of one or more glycosylation sites in human erythropoietin and the addition of one or more non-naturally occurring glycosylation sites. Erythropoietin analogs with additional glycosylation sites are disclosed in more detail in European Patent Application 640 619, to Elliot published March 1, 1995.

Furthermore, glycoproteins used in the chemical synthesis of NEAs of the present invention comprise an amino acid sequence which includes at least one additional site for glycosylation such as, but not limited to, erythropoietins comprising the sequence of human erythropoietin modified by a modification selected from the following:
Asn³⁰Thr³²;
Asn⁵¹Thr⁵³,
Asn⁵⁷Thr⁵⁹;
Asn⁶⁹;
Asn⁶⁹Thr⁷¹;
Ser⁶⁸Asn⁶⁹Thr⁷¹;
Val⁸⁷Asn⁸⁸Thr⁹⁰;
Ser⁸⁷Asn⁸⁸Thr⁹⁰;
Ser⁸⁷Asn⁸⁸Gly⁸⁹Thr⁹⁰;
Ser⁸⁷Asn⁸⁸Thr⁹⁰Thr⁹²;
Ser⁸⁷Asn⁸⁸Thr⁹⁰Ala¹⁶²;
Asn⁶⁹Thr⁷¹Ser⁸⁷Asn⁸⁸Thr⁹⁰;
Asn³⁰Thr³²Val⁸⁷Asn⁸⁸Thr⁹⁰;
Asn⁸⁹Ile⁹⁰Thr⁹¹;
Ser⁸⁷Asn⁸⁹Ile⁹⁰Thr⁹¹;
Asn¹³⁶Thr¹³⁸;
Asn¹³⁸Thr¹⁴⁰;
Thr¹²⁵; and
Pro¹²⁴Thr¹²⁵.

The notation used herein for modification of amino acid sequence means that the position(s) of the corresponding unmodified protein (e.g. hEPO of SEQ ID NO:1 or SEQ ID NO:2) indicated by the superscripted number(s) is changed to the amino acid(s) that immediately precede the respective superscripted number(s).

The glycoprotein may also be an analog having at least one additional amino acid at the carboxy terminal end of the glycoprotein, wherein the additional amino acid includes at least one glycosylation site, i.e. the conjugate as defined above also refers to a compound wherein the glycoprotein has a sequence comprising the sequence of human erythropoietin and a second sequence at the carboxy terminus of the human erythropoietin sequence, wherein the second sequence contains at least one glycosylation site. The additional amino acid may comprise a peptide fragment derived from the carboxy terminal end of human chorionic gonadotropin. Preferably, the glycoprotein is an analog selected from the group consisting of (a) human erythropoietin having the amino acid sequence, Ser Ser Ser Ser Lys Ala Pro Pro Pro Ser Leu Pro Ser Pro Ser Arg Leu Pro Gly Pro Ser Asp Thr Pro IIe Leu Pro Gln (SEQ ID NO:3), extending from the carboxy terminus; (b) the analog in (a) further comprising Ser⁸⁷ Asn⁸⁸ Thr⁹⁰ EPO; and (c) the analog in (a) further comprising Asn³⁰ Thr³² Val⁸⁷ Asn⁸⁸ Thr⁹⁰ EPO.

The glycoprotein may also be an analog having an amino acid sequence which includes a rearrangement of at least one site for glycosylation. The rearrangement may comprise a deletion of any of the N-linked carbohydrate sites in human erythropoietin and an addition of an N-linked carbohydrate site at position 88 of the amino acid sequence of human erythropoietin. Preferably, the glycoprotein is an analog selected from the group consisting of Gln⁷⁴ Ser⁸⁷ Asn⁸⁸ Thr⁹⁰ EPO; Gln³⁸ Ser⁸⁷ Asn⁸⁸ Thr⁹⁰ EPO; and Gln⁸³ Ser⁸⁷ Asn⁸⁸ Thr⁹⁰ EPO.

As used herein, "lower alkyl" means a linear or branched alkyl group having from one to six carbon atoms. Examples of lower alkyl groups include methyl, ethyl and isopropyl. In accordance with this invention, R is any lower alkyl. Conjugates in which R is methyl are preferred.

The symbol "m" represents the number of ethylene oxide residues (OCH₂CH₂) in the poly(ethylene oxide) group. A single PEG subunit of ethylene oxide has a molecular weight of about 44 daltons. Thus, the molecular weight of the conjugate (excluding the molecular weight of the EPO) depends on the number "m". In the conjugates of this invention "m" is from about 450 to about 900 (corresponding to a molecular weight of about 20 kDa to about 40 kDa), preferably from about 650 to about 750 (corresponding to a molecular weight of about 30 kDa). The number m is selected such that the resulting conjugate of this invention has a physiological activity comparable to unmodified EPO, which activity may represent the same as, more than, or a fraction of the corresponding activity of unmodified EPO. A molecular weight of "about" a certain number means that it is within a reasonable range of that number as determined by conventional analytical techniques. The number "m" is selected so that the molecular weight of each poly(ethylene glycol) group covalently linked to the erythropoietin glycoprotein is from about 20kDa to about 40kDa, and is preferably about 30kDa.

In the conjugates of this invention, the number "n" is the number of polyethylene glycol groups covalently bound to free amino groups (including ε-amino groups of a lysine amino acid and/or the amino-terminal amino group) of an erythropoietin protein *via* amide linkage(s). A conjugate of this invention may have one, two, or three PEG groups per molecule of EPO. "n" is an integer ranging from 1 to 3, preferably "n" is 1 or 2, and more preferably "n" is 1.

The compound of Formula I can be prepared from the known polymeric material: 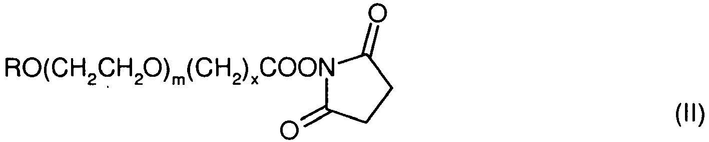 in which R and m are as described above, by condensing the compound of Formula II with the erythropoietin glycoprotein. Compounds of Formula II in which x is 3 are alpha-lower alkoxy, butyric acid succinimidyl esters of poly(ethylene glycol) (lower alkoxy-PEG-SBA). Compounds of Formula II in which x is 2 are alpha-lower alkoxy, propionic acid succinimidyl esters of poly(ethylene glycol) (lower alkoxy-PEG-SPA). Any conventional method of reacting an activated ester with an amine to form an amide can be utilized. In the reaction described above, the exemplified succinimidyl ester is a leaving group causing the amide formation. The use of succinimidyl esters such as the compounds of formula II to produce conjugates with proteins are disclosed in U.S. Patent No. 5,672,662, issued September 30, 1997 (Harris, et al.).

Human EPO contains nine free amino groups, the amino-terminal amino group plus the ε-amino groups of 8 lysine residues. When the pegylation reagent was combined with a SBA compound of Formula II, it has been found that at pH 7.5, a protein:PEG ratio of 1:3, and a reaction temperature of from 20-25°C, a mixture of mono-, di-, and trace amounts of the tri-pegylated species were produced. When the pegylation reagent was a SPA compound of Formula II, at similar conditions except that the protein:PEG ratio was 1:2, primarily the mono-pegylated species is produced. The pegylated EPO can be administered as a mixture, or as the cation exchange chromatography separated different pegylated species. By manipulating the reaction conditions (e.g., ratio of reagents, pH, temperature, protein concentration, time of reaction etc.), the relative amounts of the different pegylated species can be varied.

This invention provides the use of a composition comprised of conjugates as described above. A composition containing at least ninety percent mono-PEG conjugates, i.e. in which n is 1. can be prepared as shown in Example 5. Usually mono-PEG conjugates of erythropoietin glycoproteins are desirable because they tend to have higher activity than di-PEG conjugates. The percentage of mono-PEG conjugates as well as the ratio of mono-and di-PEG species can be controlled by pooling broader fractions around the elution peak to decrease the percentage of mono-PEG or narrower fractions to increase the percentage of mono-PEG in the composition. About ninety percent mono-PEG conjugates are a good balance of yield and activity. Sometimes compositions in which, for example, at least ninety-two percent or at least ninety-six percent of the conjugates are mono-PEG species (n equals 1) may be desired. In an embodiment of this invention the percentage of conjugates where n is I is from ninety percent to ninety-six percent.

It is counterintuitive that a chemically modified erythropoietic protein as presented in this invention would be able to pass through the BBB by simple diffusion since the NEA of the invention is highly hydrophilic and has a large molecular weight. This is because Partridge et al. (Pharmaceutical Research, Vol. 15, No. 4, 1998) have shown that pegylation with a small PEG molecule (2000 Dalton molecular weight) reduces passive brain uptake of peptides such as the brain-derived neurotrophic factor. Nevertheless, our examples below demonstrate clearly the presence of the NEA in the CSF. These findings are consistent with, and we thus hypothesize that, the NEA of the invention comprising poly(ethylene glycol) moieties integrated into the structure of the molecule pass through the BBB by a facilitated or active transport process.

Patients suffering from stroke have to be treated with the inventive NEA as soon as possible. With respect to chronic neurological diseases the NEA will be administered periodically due to its improved resident time in the blood circuit (that is, longer half-life). Since the NEA has a long resident time and shows a reduced affinity to the EPO receptor, the haemoglobin level can be controlled in a pretty narrow range. Because the peaks and troughs of the haemoglobin level that are usually found with EPO are reduced by administering the NEA, negative side effects like an increased risk of thrombosis and an unwanted thickening of the blood are reduced.

The invention is further described below by demonstrative examples. These examples portray various embodiments of the invention, but are not intended to limit the application.

### EXAMPLES

### Example 1: Pegylation of EPO with mPEG-SBA

The fermentation and purification of human EPO is e.g. described in US 6,583,272, Example 1.

EPO purified in accordance with the serum free procedure of Example 1 in US 6,583,272 (EPOsf) was homogeneous as determined by analytical methods and showed the typical isoform pattern consisting of 8 isoforms. It had a specific biological activity of 190,000 IU/mg as determined by the normocythaemic mouse assay. The pegylation reagent used was a methoxy-PEG-SBA, which is a compound of Formula II in which R is methyl; x is 3; and m is from 650 to 750 (average about 680, corresponding to an average molecular weight of about 30 kDa).

### Pegylation Reaction

To one hundred milligrams of EPOsf (9.71 ml of a 10.3 mg/ml EPOsf stock , 5.48 µmol) 10 ml of 0.1 M potassium phosphate buffer, pH, 7.5 containing 506 mg of 30kDa methoxy-PEG-SBA (16.5 µ mol) (obtained from Shearwater Polymers, Inc., Huntsville, Alabama) was added and mixed for 2h at room temperature (20-23 °C). The final protein concentration was 5 mg/ml and the protein:PEG reagent ratio was 1:3. After two hours, the reaction was stopped by adjusting the pH to 4.5 with glacial acetic acid and stored at - 20°C, until ready for purification.

### Purification

1. Conjugate Mixture: Approximately 28 ml of SP-SEPHAROSE FF (sulfo-propyl cation exchange resin) was packed into an AMICON glass column (2.2 x 7.5 cm) and equilibrated with 20 mM acetate buffer pH, 4.5 at a flow rate of 150 ml/h. Six millilitres of the reaction mixture containing 30 mg protein was diluted 5-fold with the equilibration buffer and applied onto the column. Unadsorbed materials were washed away with the buffer and the adsorbed PEG conjugate mixture was eluted from the column with 0.175 M NaCl in the equilibration buffer. Unmodified EPOsf still remaining on the column was eluted with 750 mM NaCl. Column was reequilibrated in the starting buffer. Samples were analyzed by SDS-PAGE and their degree of pegylation was determined. It was found that the 0.175M NaCl eluate contained, mono- as well as di- and trace amounts of the tri-pegylated species, whereas the 750 mM NaCl eluate contained unmodified EPOsf.
2. Di-PEG and Mono-PEG-EPOsf: The purified conjugate mixture eluted from the column in the previous step was diluted 4-fold with the buffer and reapplied onto the column and washed as described. Di-PEG-EPOsf and mono-PEG-EPOsf were separately eluted from the column with 0.1M NaCl and 0.175 M NaCl, respectively. Elution was also performed with 750mM NaCl to elute any remaining unmodified EPOsf.

Alternatively, the reaction mixture was diluted 5-fold with the acetate buffer and applied onto the SP-Sepharose column (∼0.5 mg protein/ml gel). Column was washed and adsorbed mono-PEG-EPOsf,di-PEG-EPOsf and unmodified EPOsf were eluted as described in the previous section.

### Results

PEG-EPOsf was synthesized by chemically conjugating a linear PEG molecule with a number average molecular weight of 30 kDa. PEG-EPOsf was derived from the reaction between the primary amino groups of EPOsf and the succinimidyl ester derivative of a 30 kDa PEG-butyric acid, resulting in an amide bond.

Results are summarized in Table 1. Purified conjugate mixture comprised of mono- and di-PEG-EPOsf and was free of unmodified EPOsf as determined by SDS-PAGE analysis. Conjugate mixture accounted for 23.4 mg or 78% of the starting material. Cation exchange chromatographic separation of mono- and di-PEG-EPOsf indicated that mono-to di-PEG ratio in the conjugate mixture was almost 1:1. After completion of the reaction, ratio of the individual components of Mono: Di: Unmodified were 40: 38: 20 (%). Overall yield was almost quantitative.

**Table 1. Summary of results of EPOsf pegylation**

| Sample | Protein (mg) | Yeld (%) |
|---|---|---|
| Rxn. Mix. | 30 | 100 |
| Mono- | 12.0 | 40 |
| Di- | 11.4 | 38 |
| Unmod. | 6.0 | 20 |
| Conju. Mix. | 23.4 | 78 |

### Example 2: Pegylation of EPO with mPEG-SPA

A different aliquot of the EPOsf used in Example 2 was reacted with 30 kDa methoxy-PEG-SPA (Shearwater Polymers, Inc., Huntsville, Alabama). Reaction was performed at a protein:reagent ratio of 1:2 and purification techniques were in accordance with Example 2. Primarily the mono-pegylated species was produced.

The in vivo activity of the described EPO conjugates are described in US 6,583,272, Example 4.

### Example 3: in vivo assays

The *in vivo* experiments were conducted in male Wistar rats from Charles River RCC, Füllinsdorf, Switzerland. EPO and EPO conjugate (generated according to Example 1) were both administered intravenously as a single dose of 25 µg/kg body weight into the tail vein of the rats. At the indicated time points (2 and 6 hours post injection), cerebrospinal fluid (CSF) samples were taken followed by collection of plasma (sublingual or terminal). CSF was obtained by insertion of a collection needle (0.7 x 19 mm) into the cerebellomedullary cistern (cisterna magna). CSF was drained by a silicon tubing (ID 0.5 mm) by capillary force. Using this technique, it is possible to obtain ∼0.1 ml of CSF from a rat.

### Compounds:

EPO, concentration: 1.84 mg/ml
Administration volume: 2 ml/kg body weight
Composition: aqueous buffer
EPO conjugate, concentration: 6.2 mg/ml
Administration volume: 2 ml/kg body weight
Composition: aqueous buffer

The compound concentration in the collected samples has been determined by Enzyme-Linked Immunosorbent Assay (ELISA).

### Results

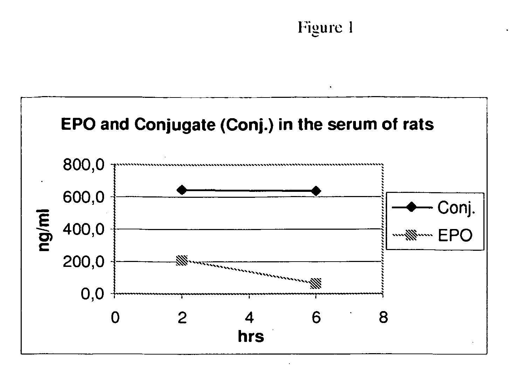

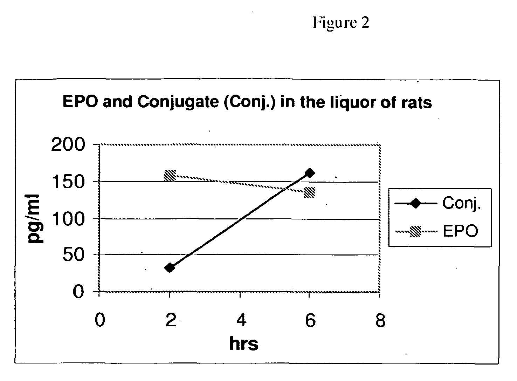

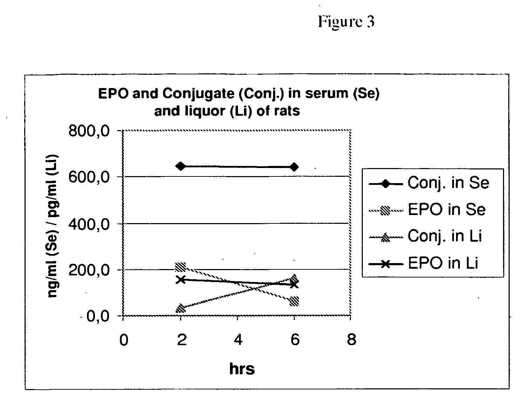

The figures 1-3 show that an NEA according to the invention is able to cross the blood brain barrier. Within the time period from 2 to 6 hours the concentration of the conjugate in the liquor increases.

## Claims

1. Use of an erythropoietic molecule for the production of a medicament for the treatment of neurodegenerative disorders of the brain and the spinal cord by administering an effective amount of the medicament in the blood circuit of a patient in need of such therapy wherein the erythropoietic molecule comprises an erythropoietin moiety having at least one free amino group selected from the group consisting of human erythropoietin and analogues thereof which have the sequence of human erythropoietin modified by the addition of from 1 to 6 glycosylation sites or a rearrangement of at least one glycosylation site; said erythropoietin moiety being covalently linked to "n" poly(ethylene glycol) groups of the formula
-CO-(CH₂)ₓ-(OCH₂CH₂)ₘ-OR
with the -CO of each poly(ethylene glycol) group forming an amide bond with one of said amino groups; wherein
R is a linear or branched alkyl group having from one to six carbon atoms;
x is 2 or 3;
m is from about 450 to about 900;
n is from 1 to 3; and
n and m are chosen so that the molecular weight of the resulting erythropoietic molecule subtracted by the molecular weight of the erythropoietic moiety is from about 20 kilodaltons to about 100 kilodaltons.

2. Use of the erythropoietic molecule according to claim 1 having the formula:
P-[NHCO-(CH₂)ₓ-(OCH₂CH₂)ₘ-OR]ₙ (I)
wherein x, m, n and R are as defined in claim 1, and P is the residue of the erythropoietin moiety without the n amino group(s) which form amide linkage(s) with the poly(ethylene glycol) group(s).

3. Use of the erythropoietic molecule according to any of the foregoing claims, wherein R is methyl.

4. Use of the erythropoietic molecule according to any of the foregoing claims, wherein m is from about 650 to about 750.

5. Use of the erythropoietic molecule according to any of the foregoing claims, wherein n is 1.

6. Use of the erythropoietic molecule according to any of the foregoing claims, wherein R is methyl; m is from about 650 to about 750; and n is 1.

7. Use of the erythropoietic molecule according to any of the foregoing claims, having the formula
[CH₃O(CH₂CH₂O)ₘCH₂CH₂CH₂CO-NH]ₙ-P
wherein m is from 650 to 750, n is 1 and P is as defined in claim 1.

8. Use of the erythropoietic molecule according to any of the foregoing claims, wherein the erythropoietin moiety is a human erythropoietin.

9. Use of the erythropoietic molecule according to any of the foregoing claims, wherein the erythropoietin moiety has the sequence SEQ ID NO: 1.

10. Use of the erythropoietic molecule according to any of the foregoing claims, wherein the erythropoietin moiety has the sequence of human erythropoietin modified by the addition of from 1 to 6 glycosylation sites.

11. Use of the erythropoietic molecule according to any of the foregoing claims wherein the neurodegenerative disorders of the brain and the spinal cord are related to an acute event selected from stroke, traumatic brain injury or spinal cord injury.

12. Use of the erythropoietic molecule according to any of the foregoing claims 1-10 wherein the neurodegenerative disorders of the brain are related to a chronic treatment and selected from schizophrenia, Alzheimer's disease, Huntington's disease, dementia, fragile X-associated tremor/ataxia syndrome, Parkinson's disease, spongiform encephalopathy, multiple sclerosis, and neurodegeneration associated with bacterial or viral infections.

13. Use of the erythropoietic molecule according to any of the foregoing claims wherein administration of the erythropoietic molecule in the blood circuit is accomplished by injection, dermal patch, subcutaneous deposit or inhalation.

14. Use of the erythropoietic molecule according to any of the foregoing claims, wherein the amount to be administered, as measured by the amount of the erythropoietin moiety, is from about 25 µg to about 500 µg/day for up to about two weeks with acute cases or from about 25 µg to about 1,000 µg/week with chronic treatment.

15. Use of the erythropoietic molecule according to claim 14 by applying 165 µg/day up to one week with acute cases or by applying 200 µg/week with chronic treatment.

16. A medicament comprising an erythropoietic molecule for use in the treatment of neurodegenerative disorders of the brain and the spinal cord by administering an effective amount of the medicament in the blood circuit of a patient in need of such therapy wherein the erythropoietic molecule comprises an erythropoietin moiety having at least one free amino group selected from the group consisting of human erythropoietin and analogues thereof which have the sequence of human erythropoietin modified by the addition of from 1 to 6 glycosylation sites or a rearrangement of at least one glycosylation site; said erythropoietin moiety being covalently linked to "n" poly(ethylene glycol) groups of the formula
-CO-(CH₂)ₓ-(OCH₂CH₂)ₘ-OR
with the -CO of each poly(ethylene glycol) group forming an amide bond with one of said amino groups; wherein
R is a linear or branched alkyl group having from one to six carbon atoms;
x is 2 or 3;
m is from about 450 to about 900;
n is from 1 to 3; and
n and m are chosen so that the molecular weight of the resulting erythropoietic molecule subtracted by the molecular weight of the erythropoietic moiety is from about 20 kilodaltons to about 100 kilodaltons.

## Patentansprüche

1. Verwendung eines erythropoetischen Moleküls zur Herstellung eines Medikaments zur Behandlung von neurodegenerativen Störungen des Hirns und des Rückenmarks durch Verabreichung einer wirksamen Menge des Medikaments in den Blutkreislauf eines Patienten, der einer solchen Therapie bedarf, wobei das erythropoetische Molekül eine Erythropoetin-Einheit mit mindestens einer freien Aminogruppe umfasst, ausgewählt aus der Gruppe bestehend aus menschlichem Erythropoetin und Analoga davon, in denen die Sequenz von menschlichem Erythropoetin durch Hinzufügen von 1 bis 6 Glycosylierungsstellen oder einer Umlagerung mindestens einer Glycosylierungsstelle modifiziert wurde; wobei die Erythropoetin-Einheit kovalent an "n" Poly(ethylenglycol)-Gruppen der Formel
-CO-(CH₂)ₓ-(OCH₂CH₂)ₘ-OR
gebunden ist, wobei die -CO-Gruppe jeder Poly(ethylenglycol)gruppe eine Amidbindung mit einer der Aminogruppen bildet; wobei
R ein linearer oder verzweigter Alkylrest mit einem bis sechs Kohlenstoffatomen ist;
x gleich 2 oder 3 ist;
m von etwa 450 bis etwa 900 ist;
n von 1 bis 3 ist; und
n und m so gewählt sind, dass das Molekulargewicht des resultierenden erythropoetischen Moleküls, abzüglich des Molekulargewichts der Erythropoetin-Einheit, von etwa 20 Kilodalton bis etwa 100 Kilodalton beträgt.

2. Verwendung des erythropoetischen Moleküls gemäß Anspruch 1 mit der Formel,
P-[NHCO-(CH₂)ₓ-(OCH₂CH₂)ₘ-OR]ₙ (I)
wobei x, m, n und R wie in Anspruch 1 definiert sind, und P der Rest der Erythropoetin-Einheit ohne die n Aminogruppe(n) ist, die (eine) Amidbindung(en) mit der/den Poly(ethylenglycol)-Gruppe(n) bildet/bilden.

3. Verwendung des erythropoetischen Moleküls gemäß einem der vorhergehenden Ansprüche, wobei R Methyl ist.

4. Verwendung des erythropoetischen Moleküls gemäß eine der vorhergehenden Ansprüche, wobei m von etwa 650 bis etwa 750 ist.

5. Verwendung des erythropoetischen Moleküls gemäß einem der vorhergehenden Ansprüche, wobei n gleich 1 ist.

6. Verwendung des erythropoetischen Moleküls gemäß einem der vorhergehenden Ansprüche, wobei R Methyl ist, m von etwa 650 bis etwa 750 ist und n gleich 1 ist.

7. Verwendung des erythropoetischen Moleküls gemäß einem der vorhergehenden Ansprüche, mit der Formel
[CH₃O(CH₂CH₂O)ₘCH₂CH₂CH₂CO-NH]ₙ-P
wobei m von 650 bis 750 ist, n gleich 1 ist und P wie in Anspruch 1 definiert ist.

8. Verwendung des erythropoetischen Moleküls gemäß einem der vorhergehenden Ansprüche, wobei die Erythropoetin-Einheit ein menschliches Erythropoetin ist.

9. Verwendung des erythropoetischen Moleküls gemäß einem der vorhergehenden Ansprüche, wobei die Erythropoetin-Einheit die Sequenz SEQ ID NO: 1 aufweist.

10. Verwendung des erythropoetischen Moleküls gemäß einem der vorhergehenden Ansprüche, wobei die Erythropoetin-Einheit die Sequenz von menschlichem Erythropoetin hat, die durch die Hinzufügung von 1 bis 6 Glykosylierungsstellen modifiziert ist.

11. Verwendung des erythropoetischen Moleküls gemäß einem der vorhergehenden Ansprüche, wobei die neurodegenerativen Störungen des Gehirns und des Rückenmarks mit einem akuten Ereignis in Zusammenhang stehen, das ausgewählt ist aus Schlaganfall, Schädel-Hirn-Trauma oder Verletzung des Rückenmarks.

12. Verwendung des erythropoetischen Moleküls gemäß einem der vorhergehenden Ansprüche 1 bis 10, wobei die neurodegenerativen Störungen des Gehirns mit einer chronischen Behandlung in Zusammenhang stehen und ausgewählt sind aus Schizophrenie, Alzheimer-Krankheit, Huntington-Krankheit, Demenz, fragile X-assoziierten Tremor/Ataxie-Syndrom, Parkinson-Krankheit, spongifonner Enzephalopathie, Multipler Sklerose, und Neurodegeneration, die mit bakteriellen oder viralen Infektionen verbunden ist.

13. Verwendung des erythropoetischen Moleküls gemäß eine der vorhergehenden Ansprüche, wobei die Verabreichung des erythropoetischen Moleküls in den Blutkreislauf durch Injektion, Hautpflaster, subkutanes Depot oder Inhalation erreicht wird.

14. Verwendung des erythropoetischen Moleküls gemäß eine der vorhergehenden Ansprüche, wobei die zu verabreichende Menge, gemessen als die Menge der Erythropoetin-Einheit, von etwa 25 µg bis etwa 500 µg/Tag für bis zu etwa zwei Wochen bei akuten Fällen beträgt oder von etwa 25 µg bis etwa 1000 µg/Woche bei chronischer Behandlung beträgt.

15. Verwendung des erythropoetischen Moleküls gemäß Anspruch 14, durch Anwenden von 165 µg/Tag für bis zu eine Woche bei akuten Fällen oder durch Anwenden von 200 µg/Woche bei chronischer Behandlung.

16. Ein Medikament umfassend ein erythropoetisches Molekül zur Verwendung in der Behandlung von neurodegenerativen Störungen des Hirns und des Rückenmarks durch Verabreichung einer wirksamen Menge des Medikaments in den Blutkreislauf eines Patienten, der einer solchen Therapie bedarf, wobei das erythropoetische Molekül eine Erythropoetin-Einheit mit mindestens einer freien Aminogruppe umfasst, ausgewählt aus der Gruppe bestehend aus menschlichem Erythropoetin und Analoga davon, in denen die Sequenz von menschlichem Erythropoetin durch die Hinzufügung von 1 bis 6 Glycosylierungsstellen oder durch Umlagerung mindestens einer Glycosylierungsstelle modifiziert wurde; wobei die Erythropoetin-Einheit kovalent "n" Poly(ethylenglycol)-Gruppen der Formel
-CO-(CH₂)ₓ-(OCH₂CH₂)ₘ-OR
gebunden ist, wobei die -CO-Gruppe jeder Poly(ethylenglycol)gruppe eine Amidbindung mit einer der Aminogruppen bildet; wobei
R ein linearer oder verzweigter Alkylrest mit einem bis sechs Kohlenstoffatomen ist;
x gleich 2 oder 3 ist;
m von etwa 450 bis etwa 900 ist;
n von 1 bis 3 ist; und
n und m so gewählt sind, dass das Molekulargewicht des resultierenden erythropoetischen Moleküls, abzüglich des Molekulargewichts der Erythropoetin-Einheit, von etwa 20 Kilodalton bis etwa 100 Kilodalton beträgt.

## Revendications

1. Utilisation d'une molécule érythropoïétique pour la production d'un médicament pour le traitement de troubles neurodégénératifs du cerveau et de la moelle épinière en administrant une quantité efficace du médicament dans le circuit sanguin d'un patient ayant besoin d'une telle thérapie, dans laquelle la molécule érythropoïétique comprend un groupement érythropoïétine ayant au moins un groupe amine libre choisi dans le groupe consistant en l'érythropoïétine humaine et ses analogues qui ont la séquence de l'érythropoïétine humaine modifiée par addition de 1 à 6 sites de glycosylation ou un réarrangement d'au moins un site de glycosylation ; ledit groupement érythropoïétine étant lié par covalence à "n" groupes poly(éthyléneglycol) de formule
-CO-(CH₂)ₓ-(OCH₂CH₂)ₘ-OR
avec le groupe -CO de chaque groupe poly(éthylène-glycol) formant une liaison amide avec un desdits groupes amino ; où
R représente un groupe alkyle linéaire ou ramifié ayant 1 à 6 atomes de carbone ;
x est égal à 2 ou 3 ;
m a une valeur d'environ 450 à environ 900 ;
n a une valeur de 1 à 3 ; et
n et m sont choisis de telle sorte que le poids moléculaire de la molécule érythropoïétique résultante soustrait du poids moléculaire du groupement érythropoïétique ait une valeur d'environ 20 kilodaltons à environ 100 kilodaltons.

2. Utilisation de la molécule érythropoïétique suivant la revendication 1, répondant à la formule :
P-[NHCO-(CH2)ₓ-(OCH₂CH₂)ₘ-OR]n (I)
dans laquelle x, m, n et R sont tels que définis dans la revendication 1 et P représente le résidu du groupement érythropoïétine sans le ou les n groupe(s) amino formant une ou plusieurs liaison(s) amide avec le ou les groupe(s) poly(éthylèneglycol);

3. Utilisation de la molécule érythropoïétique suivant l'une quelconque des revendications précédentes, dans laquelle R représente un groupe méthyle.

4. Utilisation de la molécule érythropoïétique suivant l'une quelconque des revendications précédentes, dans laquelle m a une valeur d'environ 650 à environ 750.

5. Utilisation de la molécule érythropoïétique suivant l'une quelconque des revendications précédentes, dans laquelle n est égal à 1.

6. Utilisation de la molécule érythropoïétique suivant l'une quelconque des revendications précédentes, dans laquelle R représente un groupe méthyle ; m a une valeur d'environ 650 à environ 750 ; et n est égal à 1.

7. Utilisation de la molécule érythropoïétique suivant l'une quelconque des revendications précédentes, répondant à la formule
[CH_{3O}(CH₂CH₂O) mCH₂CH₂CH₂CO-NH]ₙ-P
dans laquelle m a une valeur de 650 à 750, n est égal à 1 et P est tel que défini dans la revendication 1.

8. Utilisation de la molécule érythropoïétique suivant l'une quelconque des revendications précédentes, dans laquelle le groupement érythropoïétine est une érythropoïétine humaine.

9. Utilisation de la molécule érythropoïétique suivant l'une quelconque des revendications précédentes, dans laquelle le groupement érythropoïétine a la séquence de la SEQ ID N° 1.

10. Utilisation de la molécule érythropoïétique suivant l'une quelconque des revendications précédentes, dans laquelle le groupement érythropoïétine a la séquence de l'érythropoïétine humaine modifiée par addition de 1 à 6 sites de glycosylation.

11. Utilisation de la molécule érythropoïétique suivant l'une quelconque des revendications précédentes, dans laquelle les troubles neurodégénératifs du cerveau et de la moelle épinière sont en rapport avec un accident aigu choisi entre un ictus, une lésion cérébrale traumatique et une lésion de la moelle épinière.

12. Utilisation de la molécule érythropoïétique suivant l'une quelconque des revendications 1 à 10 précédentes, dans laquelle les troubles neurodégénératifs du cerveau sont en rapport avec un traitement chronique et sont choisis entre la schizophrénie, la maladie d'Alzheimer, la maladie de Huntington, la démence, le syndrome de tremblement/ataxie fragile associé à X, la maladie de Parkinson, l'encéphalopathie spongiforme, la sclérose en plaques et la neurodégénérescence associée à des infections bactériennes ou virales.

13. Utilisation de la molécule érythropoïétique suivant l'une quelconque des revendications précédentes, dans laquelle l'administration de la molécule érythropoïétique dans le circuit sanguin est effectuée par injection, au moyen d'un timbre dermique, par dépôt sous-cutané ou inhalation.

14. Utilisation de la molécule érythropoïétique suivant l'une quelconque des revendications précédentes, dans laquelle la quantité à administrer, telle que mesurée par la quantité du groupement érythropoïétine, va d'environ 25 µg à environ 500 µg/jour pendant une durée allant jusqu'à environ deux semaines dans des cas aigus ou d'environ 25 µg à environ 1000 µg/semaine dans le cas d'un traitement chronique.

15. Utilisation de la molécule érythropoïétique suivant la revendication 14 par application de 165 µg/jour pendant une durée allant jusqu'à une semaine dans les cas aigus ou par application de 200 µg/semaine dans le cas d'un traitement chronique.

16. Médicament comprenant une molécule érythropoïétique pour une utilisation dans le traitement de troubles neurodégénératifs du cerveau et de la moelle épinière par administration d'une quantité efficace du médicament dans le circuit sanguin d'un patient ayant besoin d'une telle thérapie, la molécule érythropoïétique comprenant un groupement érythropoïétine ayant au moins un groupe amino libre choisi dans le groupe consistant en l'érythropoïétine humaine et ses analogues qui ont la séquence de l'érythropoïétine humaine modifiée par addition de 1 à 6 sites de glycosylation ou un réarrangement d'au moins un site de glycosylation ; ledit groupement érythropoïétine étant lié par covalence à "n" groupes poly(éthylèneglycol) de formule
-CO-(CH₂)x- (OCH₂CH₂)ₘ-OR
avec le groupe -CO de chaque groupe poly(éthylène-glycol) fermant une liaison amide avec un desdits groupes amino ; où
R représente un groupe alkyle linéaire ou ramifié ayant 1 à 6 atomes de carbone ;
x est égal à 2 ou 3 ;
m a une valeur d'environ 450 à environ 900 ;
n a une valeur de 1 à 3 ; et
n et m sont choisis de telle sorte que le poids moléculaire de la molécule érythropoïétique résultante soustrait du poids moléculaire du groupement érythropoïétique ait une valeur d'environ 20 kilodaltons à environ 100 kilodaltons.
